# EUROPEAN PATENT APPLICATION

(11) **EP 4 674 982 A1**
(43) Date of publication of application: **07.01.2026**
(21) Application number: 24764137.6
(22) Date of filing: 22.02.2024
(51) Int. Cl.: C12Q 1/6886, A61P 35/00, A61P 35/04

(54) **USE OF N6-METHYLADENOSINE-MODIFIED RP11-108M9.4 FOR DIAGNOSING OR TREATING RECURRENT LIVER CANCER**

(30) Priority: 28.02.2023 KR 20230027171; 20.02.2024 KR 20240023983
(71) Applicant: Ajou University Industry-Academic Cooperation Foundation, Suwon-si, Gyeonggi-do 16499 (KR)
(72) Inventor: EUN, Jung Woo, Suwon-si, Gyeonggi-do 16223 (KR); CHEONG, Jae Youn, Suwon-si, Gyeonggi-do 16504 (KR); CHO, Hyo Jung, Suwon-si, Gyeonggi-do 16493 (KR); KIM, Soon Sun, Suwon-si, Gyeonggi-do 16517 (KR)
(74) Representative: Mewburn Ellis LLP
(86) International application number: PCT/KR2024/002316
(87) International publication number: WO 2024/181737

(57) **Abstract**

The present invention relates to use of N⁶-methyladenosine-modified RP11-108M9.4 for diagnosing or treating recurrent liver cancer. RP11-108M9.4, which is a long non-coding RNA, was presented as a marker for predicting liver cancer recurrence and verified to have the ability to regulate metastasis/recurrence. In addition, the top molecular marker of RP11-108M9.4 was identified by chemical modification. In particular, RP11-108M9.4, which is a non-coding RNA related to liver cancer recurrence, presented in the present invention, is a novel RNA molecule that has not been previously reported, and by identifying the network between cancer cells and establishing various networks of the non-coding RNA by chemical modification, a new mechanism of liver cancer occurrence and metastasis/recurrence was presented through verification in in-vitro and animal experiments. Therefore, RP11-108M9.4 discovered in the present invention is highly likely to be used as a target for treating liver cancer recurrence or metastasis.

## Description

### Technical Field

The present disclosure relates to a use of N⁶-methyladenosine-modified RP11-108M9.4 for diagnosis or treatment of a recurrent liver cancer.

### Background Art

A liver cancer has a relatively low incidence in Korea, but its relative survival rate over the past five years is 37.7%, which is the lowest survival rate along with lung cancer (34.7%) and is only half of the overall cancer survival rate of 70.7%. When it comes to factors affecting the survival rate of liver cancer patients, even with involvement of radioembolization or hepatectomy, the mortality rate reaches 21.1% and 20.4%, respectively, and the cumulative recurrence rate for two years even after treatment is 50.0% and 58.3%, respectively, indicating that the mechanisms of recurrence must be sufficiently understood and conquered to increase the survival rate of liver cancer patients. Therefore, recurrence prediction or metastasis prevention in regard to progression of liver cancer may contribute to elevation in the survival rate of liver cancer patients.

Chemical mutations in RNA may directly affect the stability and function of transcripts, and as their various roles in the homeostatic regulation of intracellular metabolism become known, they are newly receiving attention as a field of epitranscriptome. Editing and modification, which are representative RNA chemical mutations, are typically observed in cells, but have not been well understood due to scientific limitations. However, with the advancement of modern science technology, RNA mutations have been elucidated, and the roles of RNA chemical modifications such as inosine, N6-methyladenosine, 5-methylcytosine, and N1-methyladenosine have been revealed.

In particular, it has recently been discovered that the sequence-driven structure plays a crucial role for the long non-coding RNA to possess a function, and that indiscriminate chemical mutation of RNA may destroy the balance of the RNA network, causing various diseases including cancers. Attempts for multifaceted research are required to address the question of whether the regulation and function of long non-coding RNAs with abnormal expression in cancer may be affected by factors resulting from chemical mutations in RNA, thereby paving the way for a new strategy to prevent liver cancer recurrence and develop treatments therefor.

### Disclosure of the Invention

### Technical Goals

An object of the present disclosure is to provide a biomarker composition for predicting liver cancer recurrence or liver cancer metastasis or diagnosing a liver cancer, including long non-coding RNA RP11-108M9.4 as an active ingredient.

In addition, another object of the present disclosure is to provide a composition for predicting liver cancer recurrence or liver cancer metastasis or diagnosing a liver cancer including an agent capable of measuring an expression level of long non-coding RNA RP11-108M9.4 as an active ingredient; and a kit for predicting liver cancer recurrence or liver cancer metastasis or diagnosing a liver cancer including the same.

In addition, another object of the present disclosure is to provide a method of providing information necessary for predicting liver cancer recurrence or liver cancer metastasis or diagnosing a liver cancer, including measuring an expression level of long non-coding RNA RP11-108M9.4.

In addition, another object of the present disclosure is to provide a composition for preventing, attenuating, or treating liver cancer recurrence or liver cancer metastasis, including an expression or activity inhibitor of long non-coding RNA RP11-108M9.4 as an active ingredient.

In addition, another object of the present disclosure is to provide a method of screening a treatment agent for liver cancer recurrence or liver cancer metastasis through measurement of an expression level of long non-coding RNA RP11-108M9.4 in isolated liver cancer cells.

### Technical Solutions

To achieve the above objects, the present disclosure provides a biomarker composition for predicting liver cancer recurrence or liver cancer metastasis, including long non-coding RNA RP11-108M9.4 as an active ingredient.

In addition, the present disclosure provides a biomarker composition for diagnosing a liver cancer, including long non-coding RNA RP11-108M9.4 as an active ingredient.

In addition, the present disclosure provides a composition for predicting liver cancer recurrence or liver cancer metastasis, including an agent capable of measuring an expression level of long non-coding RNA RP11-108M9.4 as an active ingredient.

In addition, the present disclosure provides a composition for diagnosing a liver cancer, including an agent capable of measuring an expression level of long non-coding RNA RP11-108M9.4 as an active ingredient.

In addition, the present disclosure provides a kit for predicting liver cancer recurrence or liver cancer metastasis, including the composition.

In addition, the present disclosure provides a kit for diagnosing a liver cancer, including the composition.

In addition, the present disclosure provides a method of providing information necessary for predicting liver cancer recurrence or liver cancer metastasis, including: (1) measuring an expression level of long non-coding RP11-108M9.4 from a sample isolated from a liver cancer patient; (2) comparing the measured expression level of long non-coding RNA RP11-108M9.4 with that of a control sample; and (3) predicting that there is a high possibility of liver cancer recurrence or liver cancer metastasis if the measured expression level of long non-coding RNA RP11-108M9.4 is higher than that of the control sample.

In addition, the present disclosure provides a method of providing information necessary for diagnosing a liver cancer, including: (1) measuring an expression level of long non-coding RP11-108M9.4 from a sample isolated from a liver cancer patient; (2) comparing the measured expression level of long non-coding RNA RP11-108M9.4 with that of a control sample; and (3) determining as a liver cancer if the measured expression level of long non-coding RNA RP11-108M9.4 is higher than that of the control sample.

In addition, the present disclosure provides a pharmaceutical composition for preventing or treating liver cancer recurrence or liver cancer metastasis, including an expression or activity inhibitor of long non-coding RNA RP11-108M9.4 as an active ingredient.

In addition, the present disclosure provides a health functional food composition for preventing or attenuating liver cancer recurrence or liver cancer metastasis, including an expression or activity inhibitor of long non-coding RNA RP11-108M9.4 as an active ingredient.

In addition, the present disclosure provides a method of screening a treatment agent for liver cancer recurrence or liver cancer metastasis, including: (1) contacting isolated liver cancer cells with a test substance; (2) measuring an expression or activity level of long non-coding RNA RP11-108M9.4 in the liver cancer cells that have come into contact with the test substance; and (3) selecting a test substance having a reduced expression or activity level of the long non-coding RNA RP11-108M9.4 compared to a control sample.

### Advantageous Effects

The present disclosure relates to a use of N⁶-methyladenosine-modified RP11-108M9.4 for diagnosis or treatment of a recurrent liver cancer, wherein RP11-108M9.4, a long non-coding RNA, has been presented as a marker for predicting liver cancer recurrence and is subjected to verification on its ability to regulate metastasis/recurrence. Additionally, the upper-level molecular markers of RP11-108M9.4 were specified by chemical modification. In particular, RP11-108M9.4, which is a non-coding RNA associated with liver cancer recurrence as presented in the present disclosure, is a novel RNA molecule that has not been previously reported to be used to clarify the network between cancer cells and establish a diverse network of non-coding RNAs through chemical modification, thereby presenting a new mechanism of liver cancer occurrence and metastasis/recurrence through verification in vitro and in animal experiments. Accordingly, RP11-108M9.4 discovered herein is highly likely utilized as a target for treating recurrence or metastasis of a liver cancer.

### Brief Description of Drawings

FIG. 1 shows results of screening RP11-108M9.4, a long non-coding RNA associated with a recurrent liver cancer, using a genome DB.
FIG. 2 shows results of verifying expression and growth controllability of RP11-108M9.4.
FIG. 3 shows results of wound healing assay, cell migration assay, and cell infiltration assay conducted to determine whether RP11-108M9.4 is involved in liver cancer cell metastasis.
FIG. 4 shows results of identifying a role of RP11-108M9.4 in an EMT cell program that boosts tumorigenic and metastatic abilities of cancer cells and allows them to have resistance to various treatment regimens.
FIG. 5 shows results of verifying cancer cell controllability of RP11-108M9.4 using a mouse subcutaneous transplantation model.
FIG. 6 shows results of verifying metastasis controllability of RP11-108M9.4 in a mouse model with lung metastasis.
FIG. 7 shows results of verifying recurrence controllability of RP11-108M9.4 in a mouse orthotopic model.
FIG. 8 shows results of evaluating an impact on liver cancer recurrence using an orthotopic model, as it is expected to contribute to liver cancer recurrence.
FIG. 9 shows results of identifying markers involved in EMT via immunochemical staining of liver tissues treated with siRP11-108M9.4.
FIG. 10 shows results of upregulated expression of RP11-108M9.4 by N⁶-methyladenosine chemical mutation.
FIG. 11 shows results of identifying expression of RP11-108M9.4 and METTL3 by qRT-PCR using liver cancer tissues and surrounding tissues of a liver cancer patient who underwent hepatectomy, provided by the Human Genome Resource Center of Ajou University Hospital.
FIG. 12 shows a result of identifying expression of RP11-108M9.4 in exosomes in the Exocarta database.
FIG. 13 shows a result of identifying an expression level of RP11-108M9.4 in normal liver cell lines and liver cancer cell lines.
FIG. 14 shows results of identifying staining where a green staining area of CD81 antibody, an exosome marker, overlaps with a red staining area of EEA1 antibody, an endocytosis marker, following culture of a normal liver cell line with treatment of liver cancer-derived exosomes.
FIG. 15 shows results of observing an expression level of RP11-108M9.4 by treating EVs extracted from a liver cancer cell line SNU449 to a normal liver cell line MIHA.
FIG. 16 shows results of determining whether RP11-108M9.4 expression in EVs derived from patient serum is higher in a liver cancer as in tissues.
FIG. 17 shows results of observing a survival rate depending on survival and recurrence of liver cancer patients to evaluate the clinical significance of an expression level of EV-RP11-108M9.4.

### Best Mode for Carrying Out the Invention

The present disclosure provides a biomarker composition for predicting liver cancer recurrence or liver cancer metastasis, including long non-coding RNA RP11-108M9.4 as an active ingredient.

In addition, the present disclosure provides a biomarker composition for diagnosing a liver cancer, including long non-coding RNA RP11-108M9.4 as an active ingredient.

Preferably, a transcript of the long non-coding RNA RP11-108M9.4 may include, but is not limited to, a GGAC motif, which is a variant motif of N⁶-methyladenosine.

The term "prediction" as used herein refers to a process of predicting a treatment outcome of a pathological condition by collecting data on the progress status and the treatment process of the pathological condition. For the purpose of the present disclosure, the prediction may be interpreted as determining the probability of recurrence or metastasis following liver cancer treatment, or the possibility of death thereby, but is not limited thereto.

The term "diagnosis" as used herein includes determining the susceptibility of a subject to a particular disease or condition, determining whether a subject currently has a particular disease or condition, determining the prognosis of a subject having a particular disease or condition, or therametrics (e.g., monitoring the condition of a subject to provide information on the treatment efficacy).

In addition, the present disclosure provides a composition for predicting liver cancer recurrence or liver cancer metastasis, including an agent capable of measuring an expression level of long non-coding RNA RP11-108M9.4 as an active ingredient.

In addition, the present disclosure provides a composition for diagnosing a liver cancer, including an agent capable of measuring an expression level of long non-coding RNA RP11-108M9.4 as an active ingredient.

Preferably, the agent capable of measuring the expression level of the long non-coding RNA RP11-108M9.4 may be, but is not limited to, a primer or probe specifically binding to the long non-coding RNA RP11-108M9.4.

Preferably, the liver cancer metastasis may be, but is not limited to, metastasis from a liver cancer to a lung cancer.

In addition, the present disclosure provides a kit for predicting liver cancer recurrence or liver cancer metastasis, including the composition.

In addition, the present disclosure provides a kit for diagnosing a liver cancer, including the composition.

The term "primer" as used herein refers to a short nucleic acid sequence with a short free 3' hydroxyl group, capable of forming base pairs with a complementary template and serving as a starting point for duplication of the template strand. The primer may initiate DNA synthesis in the presence of a reagent for polymerization (i.e., DNA polymerase or reverse transcriptase) and four different nucleoside triphosphates in an appropriate buffer and temperature. PCR conditions and lengths of sense and antisense primers may be appropriately selected according to techniques known in the art.

The term "probe" as used herein refers to a nucleic acid fragment, such as RNA or DNA, that ranges from a few bases to several hundred bases in length, with capability of specifically binding to mRNA while enabling determination of the presence or absence of a specific mRNA and the expression level as it is labeled. Probes may be prepared in the form of oligonucleotide probes, single strand DNA probes, double strand DNA probes, and RNA probes. Selection of an appropriate probe and hybridization conditions may be appropriately selected according to techniques known in the art.

In addition, the present disclosure provides a method of providing information necessary for predicting liver cancer recurrence or liver cancer metastasis, including (1) measuring an expression level of long non-coding RP11-108M9.4 from a sample isolated from a liver cancer patient; (2) comparing the measured expression level of long non-coding RNA RP11-108M9.4 with that of a control sample; and (3) predicting that there is a high possibility of liver cancer recurrence or liver cancer metastasis if the measured expression level of long non-coding RNA RP11-108M9.4 is higher than that of the control sample.

In addition, the present disclosure provides a method of providing information necessary for diagnosing a liver cancer, including (1) measuring an expression level of long non-coding RP11-108M9.4 from a sample isolated from a liver cancer patient; (2) comparing the measured expression level of long non-coding RNA RP11-108M9.4 with that of a control sample; and (3) determining as a liver cancer if the measured expression level of long non-coding RNA RP11-108M9.4 is higher than that of the control sample.

Preferably, the increase in the expression level of the long non-coding RNA RP11-108M9.4 may be induced by, but is not limited to, mutation in N6-methyladenosine of RP11-108M9.4 by METTL3.

Specifically, the method of measuring the expression level of the long non-coding RNA RP11-108M9.4 may involve the use of, but is not limited to, RT-PCR, competitive RT-PCR, real-time RT-PCR, RNase protection assay (RPA), Northern blotting, DNA chip, Western blot, enzyme linked immunosorbent assay (ELISA), radioimmunoassay (RIA), radioimmunodiffusion, Ouchterlony immunodiffusion, rocket immunoelectrophoresis, histoimmunostaining, immunoprecipitation assay, complement fixation assay, FACS, or protein chip.

The term "sample isolated from a patient" as used herein includes, but is not limited to, samples such as tissue, cells, whole blood, serum, plasma, saliva, sputum, cerebrospinal fluid, or urine that differ from a control in terms of expression levels of the long non-coding RNA RP11-108M9.4, which is a biomarker of the present disclosure. More preferably, in the present disclosure, the sample may be a serum exosome in relation to the diagnosis of a liver cancer.

In addition, the present disclosure provides a pharmaceutical composition for preventing or treating liver cancer recurrence or liver cancer metastasis, including an expression or activity inhibitor of long non-coding RNA RP11-108M9.4 as an active ingredient.

Preferably, the expression or activity inhibitor of the long non-coding RNA RP11-108M9.4 may be selected from the group consisting of antisense nucleotides, small interfering RNA (siRNA), short hairpin RNA (shRNA), compounds, peptides, aptamers, and antibodies that specifically bind to RP11-108M9.4, but is not limited thereto.

Preferably, the liver cancer metastasis may be, but is not limited to, metastasis from a liver cancer to a lung cancer.

The pharmaceutical composition of the present disclosure may be prepared using pharmaceutically suitable and physiologically acceptable adjuvants in addition to the active ingredient, and solubilizing agents such as excipients, disintegrants, sweeteners, binders, coating agents, swelling agents, lubricants, glidants, or flavoring agents may be used as the adjuvant. The pharmaceutical composition of the present disclosure may be preferably formulated as a pharmaceutical composition by additionally including one or more pharmaceutically acceptable carriers in addition to the active ingredient for administration. In the composition formulated as a liquid solution, the acceptable pharmaceutical carriers are those that are sterile and biocompatible, such as saline solution, sterile water, Ringer's solution, buffered saline, albumin injection solution, dextrose solution, maltodextrin solution, glycerol, ethanol, and mixtures of one or more of these components, and other conventional additives, such as antioxidants, buffers, and bacteriostatic agents, may be added as necessary. In addition, diluents, dispersants, surfactants, binders, and lubricants may be additionally added to formulate the composition into injectable formulations such as aqueous solutions, suspensions, and emulsions, pills, capsules, granules, or tablets.

The pharmaceutical formulation form of the pharmaceutical composition of the present disclosure may be a granule, powder, coated tablet, tablet, capsule, suppository, syrup, juice, suspension, emulsion, drop or injectable liquid, and a sustained-release formulation of the active compound. The pharmaceutical composition of the present disclosure may be administered in a conventional manner via intravenous, intraarterial, intraperitoneal, intramuscular, intraarterial, intraperitoneal, intrasternal, transdermal, intranasal, inhalation, topical, rectal, oral, intraocular, or intradermal routes. The effective amount of the active ingredient of the pharmaceutical composition of the present disclosure refers to an amount required for prevention or treatment of a disease. Therefore, it may be adjusted according to various factors including the type of disease, the severity of the disease, the type and content of the active ingredient and other ingredients contained in the composition, the type of formulation, and the patient's age, weight, general health status, sex and diet, administration time, administration route and secretion rate of the composition, treatment period, and concomitant drugs. Though not limited thereto, for example, in the case of adults, when administered once to several times a day, the composition of the present disclosure may be administered at a dose of 0.1 ng/kg to 10 g/kg in the case of a compound, 0.1 ng/kg to 10 g/kg in the case of a polypeptide, protein, or antibody, and 0.01 ng/kg to 10 g/kg in the case of an antisense nucleotide, siRNA, shRNA, or miRNA, when administered once to several times a day.

In addition, the present disclosure provides a health functional food composition for preventing or attenuating liver cancer recurrence or liver cancer metastasis, including an expression or activity inhibitor of long non-coding RNA RP11-108M9.4 as an active ingredient.

The health functional food composition of the present disclosure may be provided in the form of powder, granules, tablets, capsules, syrup, or beverage, and the health functional food composition may be used together with other foods or food additives in addition to the active ingredient and may be used appropriately according to a conventional method. The amount of active ingredients mixed may be appropriately determined depending on its intended use, for example, preventive, health, or therapeutic treatment.

The effective dose of the active ingredient contained in the health functional food composition may be used in accordance with the effective dose of the pharmaceutical composition, but in the case of long-term intake for the purpose of health and hygiene or health care, it may be below the range, and it is certain that it may be used in an amount exceeding the range since the active ingredient has no problems in terms of safety.

There are no special limitations on the types of the health foods, and examples include meat, sausage, bread, chocolate, candy, snacks, confectionery, pizza, ramen, other noodles, gum, dairy products including ice cream, various soups, beverages, tea, drinks, alcoholic beverages, and vitamin complexes.

In addition, the present disclosure provides a method of screening a treatment agent for liver cancer recurrence or liver cancer metastasis, including (1) contacting isolated liver cancer cells with a test substance; (2) measuring an expression or activity level of long non-coding RNA RP11-108M9.4 in the liver cancer cells that have come into contact with the test substance; and (3) selecting a test substance having a reduced expression or activity level of the long non-coding RNA RP11-108M9.4 compared to a control sample.

The term "test substance" used in referring to the screening method of the present disclosure refers to an unknown candidate substance used in screening to examine whether it affects the expression level of a gene or the expression or activity of a protein. The sample includes, but is not limited to, chemicals, nucleotides, antisense RNA, small interference RNA (siRNA), and natural product extracts.

### Modes for Carrying Out the Invention

Hereinafter, the present disclosure will be described in detail according to examples that do not limit the present disclosure. It should be noted that the following examples of the present disclosure are only intended to illustrate the present disclosure and do not limit or restrict the scope of the present disclosure. Therefore, what a skilled person in the art to which the present disclosure pertains may easily infer from the detailed description and examples of the present disclosure is interpreted as falling within the scope of the present disclosure.

### <Experimental Example>

The following Experimental Examples are intended to provide Experimental Examples commonly applied to each example according to the present disclosure.

### 1. Screening of recurrence-specific long non-coding RNA indicators from whole transcriptome data of recurrent liver diseases

To select long non-coding RNAs that are indicators of liver cancer recurrence, an analysis of recurrence indicators using GSE114564, a multi-stage liver disease and liver cancer data that may identify recurrence clinical information, as well as TCGA (The Cancer Genome Atlas), which is a public omics data. Heatmap and Venn diagram techniques were used to identify long non-coding RNAs associated with recurrence.

### 2. Collection of tissues, blood, and clinical information from a cohort of liver diseases for validation

The study was conducted with approval from the Institutional Review Board (IRB) of Ajou University Hospital, using 86 pairs of liver cancer tissues (liver cancer tissues and surrounding non-liver cancer tissues) from the Human Biobank, sera from the normal, liver disease, and liver cancer cohorts, and clinical information from corresponding patients.

### 3. RNA extraction from tissues

Tissues frozen in liquid nitrogen were thoroughly crushed in a bowl, and RNA was extracted by treating QIAzol Lysis Reagent (Cat#79306).

### 4. Measurement of an expression level of RP11-108M9.4 by qRT-PCR analysis

cDNA synthesis was performed using PrimeScript^{™} RT Master Mix (Perfect Real Time) (Cat #RR036A) (TaKaRa).

Tissue RNA and RNase free water were combined to make 8 ul, and 2 ul of PrimeScript^{™} RT Master Mix was added to make a total of 10 ul for cDNA synthesis.

cDNA conditions were set as follows.
(When using PrimeScript RT master mix)
A. Stage 1: 37°C, 15 minutes
B. Stage 2: 85°C, 5 seconds later 4°C, -ing

qRT-PCR was performed using primers with the sequences below. The primers used at this time were purchased from M.biotech (Hanam, Korea). qRT-PCR was performed using 5ul of qPCR Master Mix (2X, High ROX) (Gendepot, Cat #Q5602) in a total volume of 10ul.

**TABLE 1**

| Gene | Accession No. | Forward sequence | Reverse sequence |
|---|---|---|---|
| RP11-108 M9.4 | ENST000004221 24.1 | 5'-ACCTGAAAGAGCCCCA AACT-3' | 5'-AAGCAAACAATGG GAGCAAG-3' |
| METTL3 | NM_019852.5 | 5'-GAGTGCATGAAAGCCA GTGA-3' | 5'-ACTGGAATCACCTC CGACAC-3' |
| GAPDH | NM_002046.6 | 5'-AGTATGACAACAGCCT CAAG-3' | 5'-TCATGAGTCCTTCC ACGATA-3' |
| HMBS | NM_001024382. 2 | 5'-GGAGGGCAGAAGGAAG AAAACAG-3' | 5'-CACTGTCCGTCTGT ATGCGAG-3' |

qRT-PCR conditions were set as follows.
A. Stage 1: 95°C, 2 minutes
B. Stage 2: 95°C, 15 seconds
C. : 58-60°C, 34 seconds
D. : 72°C, 30 seconds
E. Repeat 45 cycles of Stage 2

### 5. Cell culture

Human HCC cell lines (Huh-7, SNU449, PLC/PRF/5) used were obtained from the Korean Cell Line Bank (Seoul, Korea). Each cell line was cultured in DMEM containing 10% FBS and 100 units/mL penicillin-streptomycin (Invitrogen, CA, USA) and RPMI1640 medium. All cultures were carried out under conditions where 5% carbon dioxide and a temperature of 37°C are maintained.

### 6. Transfection

siRNAs used for transfection were synthesized by Genolution (Seoul, Korea). The base sequences of siRNA are shown in Table 2 below.

**TABLE 2**

| RNA duplexes | Sense sequence |
|---|---|
| Control siRNA | 5'-UUCUCCGAACGUGUCACGUUU-3' |
| RP11-108M9.4 siRNA | 5'-GGACUUGCUCCCAUUGUUUUU-3' |
| METTL3 siRNA | 5'-GCAGGUUCAACAUACCCGUUU-3' |

Transfection was performed using Lipofectamine 2000 (Invitrogen) reagent according to the protocol provided by the manufacturer.

### 7. Analysis of cell growth

For cell growth analysis, cells were first seeded into a 24-well plate to about 30% confluence, transfected, and then treated with 0.5 mg/mL 3-(4,5-dimethylthiazol-2-yl)-2,5-diphenyltetrazolium bromide (MTT), followed by a reaction for 1 hour. Formazan crystals were dissolved in DMSO, and the absorbance was measured at 570 nm.

### 8. Wound healing assay

Transfected cells were seeded into 6-well plates, and when reaching 90-100% confluency, the cells were scratched using a micropipette tip. Afterwards, the image of the area with the scratches was observed through a microscope.

### 9. Cell infiltration assay

Cell infiltration assay was performed by coating Matrigel (BD Bioscience).

For infiltration assay, Matrigel was diluted to a concentration of 0.3 mg/mL, dispensed into each insert by 100 µl each, and cultured at 37°C for 2 hours to spread the cells transfected with siRP11-108M9.4 to the insert, followed by culture in a medium containing 2.5~5% FBS as a chemoattractant.

After the time conditions set for each cell had elapsed, the infiltrating cells were stained and observed.

### 10. Preparation of a mouse model with liver cancer transplantation

### 1) Subcutaneous transplantation models

For xenograft tumorgenesis assays, 5 × 10⁵ transfected cells were mixed with serum free media and 20% Matrigel, and the cell suspension was injected subcutaneously into 6-week-old BALB/c nude mice.

Observation was followed three times a week on whether the tumor is generated at the injection site in the mice. Each experimental group consisted of six mice, and tumor growth was measured in three orthogonal directions using calipers.

Tumor volume was calculated as 0.5 × length (L) × width² (W²).

### 2) Tail vein injection models

For the tail vein injection model to identify the metastatic ability of liver cancer cells, 1.5 × 10⁶ transfected cells were mixed with serum free media, and the cell suspension was injected into the tail vein of 6-week-old athymic mice once every 3 weeks.

The body weights of the mice were measured twice a week, and 10 weeks after the first injection, the mice were sacrificed to check for liver cancer cells that had metastasized to the lungs.

### 3) Orthotopic transplantation models

To identify the recurrent ability of liver cells, the skin and peritoneum of the mouse were incised to expose the left lobe of the liver outside the body, and then 5 × 10⁵ transfected cells were mixed with serum free media and 50% Matrigel, followed by injection of the cell suspension into the left lobe of the liver of 6-week-old BALB/c nude mice.

Two weeks later, the upper abdominal wall of the mouse was resected to expose the left lobe of the liver into which the cell suspension had been injected to check tumor formation, the area of the liver where the tumor had formed was resected, the left lobe of the liver was then placed back into the body, and the incision site was sutured.

Twelve days later, the mice were sacrificed, and the number of tumors recurring in the left lobe of the liver as well as the size and weight of the tumors were determined.

### 11. Identification of chemical modifications of RNA by immunoprecipitation

To determine whether the N⁶-methyladenosine mutation exists on the RP11-108M9.4 transcript, RNA immunoprecipitation was performed, targeting RP11-108M9.4 using the Magna RIP RNA-Binding Protein Immunoprecipitation Kit (Merck, Cat#17-700).

After collecting the liver cancer cell line washed with cold PBS, the cells were purified with a centrifuge, and RIP lysis buffer was added. While the cells were disrupted, 5 µg of Rabbit IgG (Merck, Cat#PP64B), m⁶A antibody (Synaptic Systems, Cat#202 003), and METTL3 antibody (abcam, Cat#195352) were added to a tube containing the magnetic beads washed with RIP wash buffer, followed by a reaction for 30 minutes at room temperature. Using a magnetic separator (Sigma, Cat#20-400), only the antibody-magnetic bead complex was purified, and the lysate of the liver cancer cell line disrupted in RIP immunoprecipitation buffer (RIP wash buffer, 0.5 M EDTA, RNase inhibitor) was added. After reaction at 4°C overnight, the supernatant was discarded using a magnetic separator and washed six times with RIP wash buffer.

After the final washing, 150 µl of Proteinase K Buffer (RIP Wash Buffer, 10% SDS, Proteinase K) was added and reacted at 55°C for 30 minutes. Afterwards, the supernatant excluding the beads was transferred to a new tube through a magnetic separator, RNA was extracted to synthesize cDNA, and qRT-PCR was performed.

### 12. EV delivery-IF

To check the cellular uptake of EVs, normal liver cell line (MIHA) was treated with liver cancer-derived exosomes, and then immunofluorescence staining was performed using CD81 antibody, an exosome marker, in green, EEA1 antibody, an endocytosis marker, in red, and cell nuclei in blue using DAPI, and then images were taken using a fluorescence microscope.

### 13. Exosome isolation and RNA extraction from patient serum

### 1) Isolation of exosomes from patient serum

72ul of ExoQuick^{™} was added to 300ul of patient serum, vortexed, and stored at 4°C for one day. Exosome extraction was detected using pellets generated by centrifugation at 13,000 rpm and 4°C for 2 minutes.

### 2) RNA extraction from isolated exosomes

After adding 350 ul of lysis buffer to the pellet, pooling was conducted by pipetting, followed by incubation at RT for 5 minutes. After adding 200 ul of EtOH, vertexing was performed for 10 seconds. After preparing the spin column and collection tube and then transferring the entire sample to the column, centrifugation was performed at 13,000 rpm for 1 minute at room temperature to remove the solution that moved down to the collection tube, 400 ul of wash buffer was added followed by centrifugation at 13,000 rpm for 1 minute at room temperature for removal of the solution that moved down to the collection tube, after which the process was repeated and then centrifugation was performed at 13,000 rpm for 2 minutes for final drying.

Instead of the collection tube, the spin column was combined with the e-tube, 30 ul of elution buffer was added, centrifugation was performed at 2,000 rpm for 2 minutes, and then the final centrifugation was carried out at 13,000 rpm for 1 minute, which was repeated twice. The concentration of the finally acquired solution was measured using Nano Drop.

14. Measurement of expression levels in serum and serum exosomes of liver disease cohort patients via qRT-PCR analysis

cDNA synthesis was performed using PrimeScript^{™} RT Master Mix (Perfect Real Time) (Cat #RR036A) (TaKaRa), and miScript RT II kit (Cat #218161) (QIAGEN).

Tissue RNA and RNA free water were combined to make 8 ul, and 2 ul of PrimeScript^{™} RT Master Mix was added to make a total of 10 ul for cDNA synthesis.

Serum exosome RNA and RNA free water (RFW) were combined to make 12 ul, and 8 ul of miScript RT II Buffer (4 ul of 5X miScript Hiflex buffer, 2 ul of 10X miScript Nucleics Mix, 2 ul of miScript Reverse TranScriptase Mix) was added to synthesize cDNA in a total of 20 ul.

cDNA conditions were set as follows.
(When using PrimeScript RT master mix)
   A. Stage 1: 37°C, 15 minutes
   B. Stage 2: 85°C, 5 seconds later 4°C, -ing
(When using miScript RT II kit)
   A. Stage 1: 37°C, 60 minutes
   B. Stage 2: 95°C, 5 seconds later 4°C, -ing

### <Example 1> Selection of RP11-108M9.4, a long non-coding RNA associated with a recurrent liver cancer, using genome DB

To select a long non-coding RNA specific for liver cancer recurrence, analysis was conducted on Catholic_LIHC and TCGA_LIHC which are liver cancer genomic data containing clinical information on recurrence, and RP11-108M9.4, a long non-coding RNA specific for liver cancer recurrence, was selected.

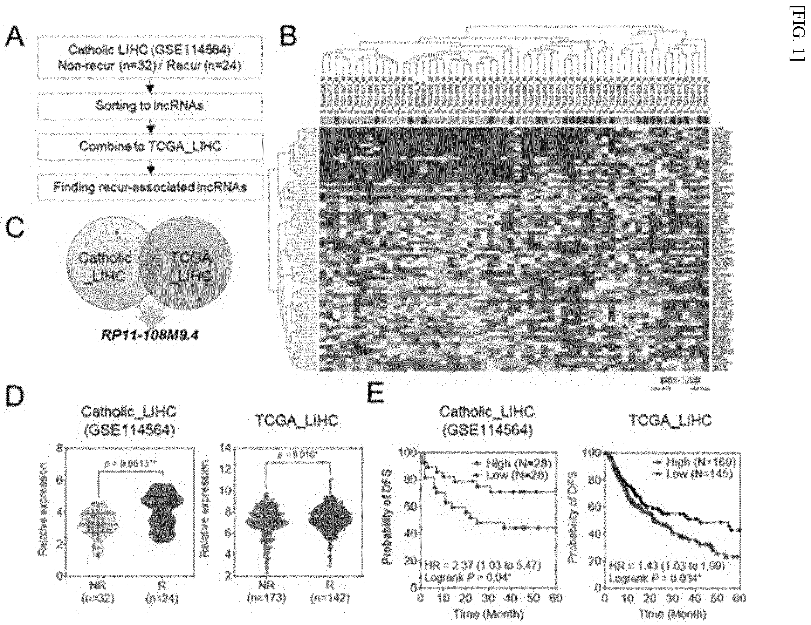

RP11-108M9.4 expression was more increased in the recurrent liver cancer (R, recur) group than in the non-recurrent liver cancer (NR, non-recur) group, and high expression of RP11-108M9.4 was associated with a poor prognosis of liver cancer patients (FIG. 1).

### <Example 2> Verification of expression and growth controllability of RP11-108M9.4

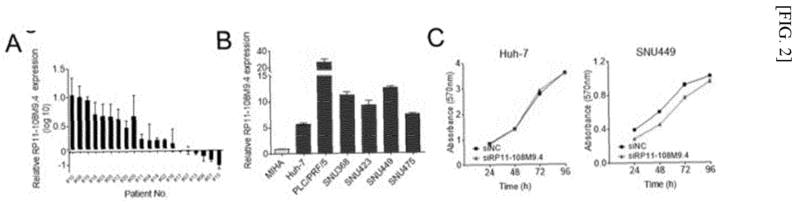

The expression of RP11-108M9.4 was generally increased in liver cancer tissues and liver cancer cell lines, and when RP11-108M9.4 was knocked down and MTT was performed to determine whether it affects liver cancer growth, it was found that it had no effect on the growth of liver cancer cells (FIG. 2).

### <Example 3> Verification of control function of RP11-108M9.4 for liver cancer metastasis

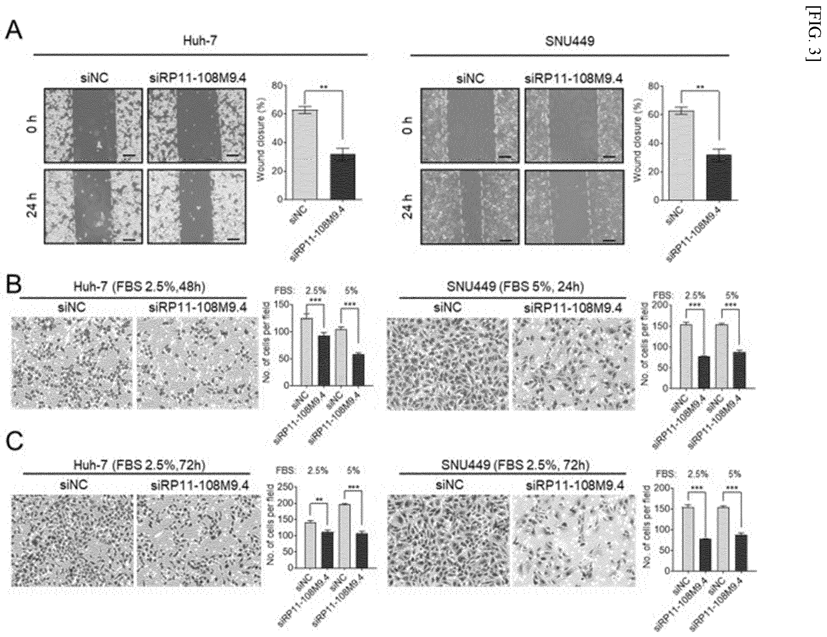

To determine whether RP11-108M9.4 is involved in liver cancer cell metastasis, wound healing assay, cell migration assay, and cell infiltration assay were performed. In contrast to the fact that there was no correlation with the proliferation of liver cancer cells, when the expression of RP11-108M9.4 was reduced, wound healing ability, migration ability, and infiltration ability were shown to be reduced compared to the experimental control group (FIG. 3).

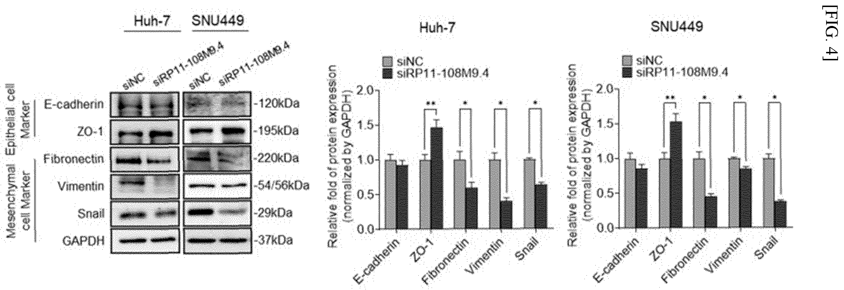

It was attempted to determine the role of RP11-108M9.4 in the EMT cell program, which enhances the tumorigenic and metastatic abilities of cancer cells and makes them resistant to various treatment regimens. Similarly, when the expression of RP11-108M9.4 was reduced and the protein expression of epithelial cell markers E-cadherin and ZO-1 was identified over the experimental control group, it was found that ZO-1 was significantly increased in both Huh-7 and SNU449, and when the mesenchymal cell markers fibronectin, vimentin, and snail were identified, they were significantly decreased in both cell lines, such that it is considered that RP11-108M9.4 is involved in EMT signaling in hepatocellular carcinoma to target the molecular mechanism of tumor suppression when it is inhibited. (FIG. 4).

### <Example 4> Verification of cancer cell controllability of RP11-108M9.4 using mouse subcutaneous transplantation model

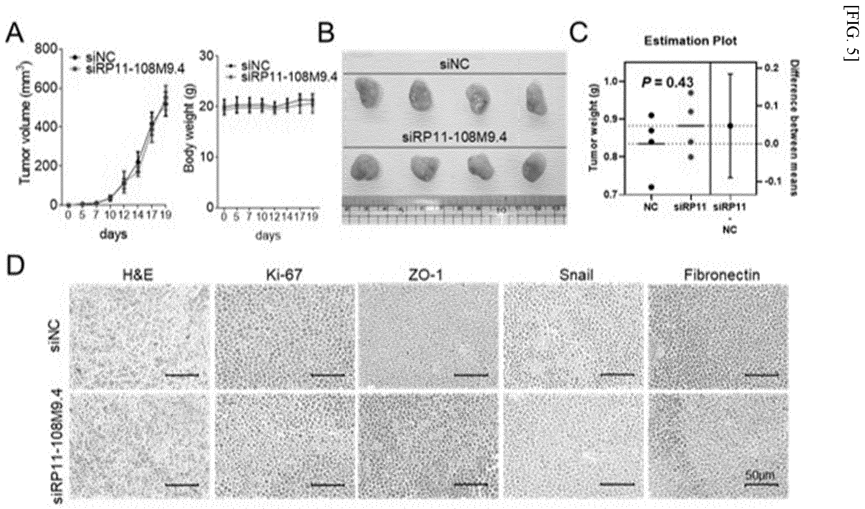

As a result of observing tumor formation in mice subcutaneously injected with Huh-7 liver cancer cell lines with reduced expression of RP11-108M9.4, no changes in tumor size or weight were observed, which is the same outcome as the test tube experiment. Even when determined by the immunohistochemistry, no change was observed in Ki-67, an indicator of cell growth, but the expression of ZO-1, a marker for metastatic ability of cancer cells, increased while the expression of snail and fibronectin actually decreased (FIG. 5).

### <Example 5> Verification of metastasis controllability of RP11-108M9.4 in a mouse lung metastasis models

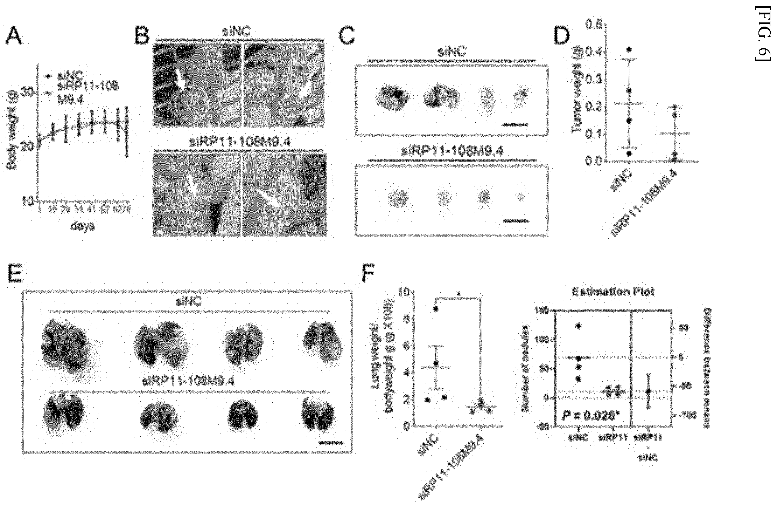

When the metastasis controllability of RP11-108M9.4 was identified using a mouse lung metastasis model, the experimental group that received tail vein injection of Huh-7 with reduced expression of RP11-108M9.4 showed a decrease in lung nodules and a further decrease in lymph node metastasis, showing the liver cancer metastasis controllability of RP11-108M9.4 (FIG. 6).

### <Example 6> Verification of recurrence controllability of RP11-108M9.4 in mouse orthotopic models

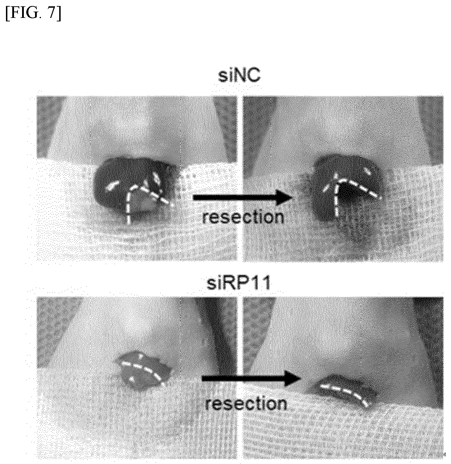

RP11-108M9.4 showed higher expression in patients with the recurrent liver cancer and was expected to contribute to liver cancer recurrence, such that its effect on liver cancer recurrence was evaluated using an orthotopic model. Huh-7 cell lines treated with siNC and siRP11-108M9.4 were injected into the liver of mice to induce liver cancer, and then partial resection was performed to evaluate the recurrence of liver cancer (FIG. 7).

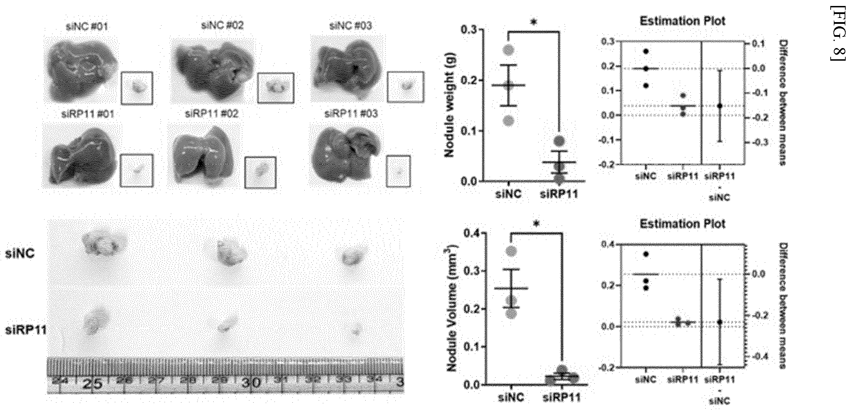

In mice treated with siRP11-108M9.4, the size and weight of relapsed liver cancer were shown to be significantly reduced compared to siNC (FIG. 8).

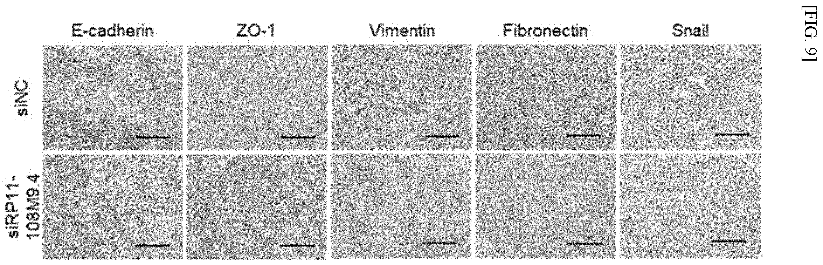

In addition, when markers involved in EMT were identified using immunochemical staining of liver tissues treated with siRP11-108M9.4, it was found that ZO-1 increased as the test tube results, and when mesenchymal cell markers fibronectin, vimentin, and snail were identified, they were decreased, observing the same results in vitro and in vivo (FIG. 9).

### <Example 7> Upregulated expression control of RP11-108M9.4 by N6-methyladenosine chemical mutation

When the transcript sequence of RP11-108M9.4 was identified, it was found that there were three GGAC motifs, which are mutant motifs of N⁶-methyladenosine, even though the transcript is relatively small in size, consisting of 326 nucleotides. In other words, the increased expression of RP11-108M9.4 in the liver cancer could be induced by the N⁶-methyladenosine mutation made in the GGAC motif.

When four large-scale liver cancer transcriptome data was examined, it was found that the expression of METTL3, a factor that induces N⁶-methyladenosine, was increased, and when the expression of METTL3 was decreased or increased, the expression of RP11-108M9.4 was regulated.

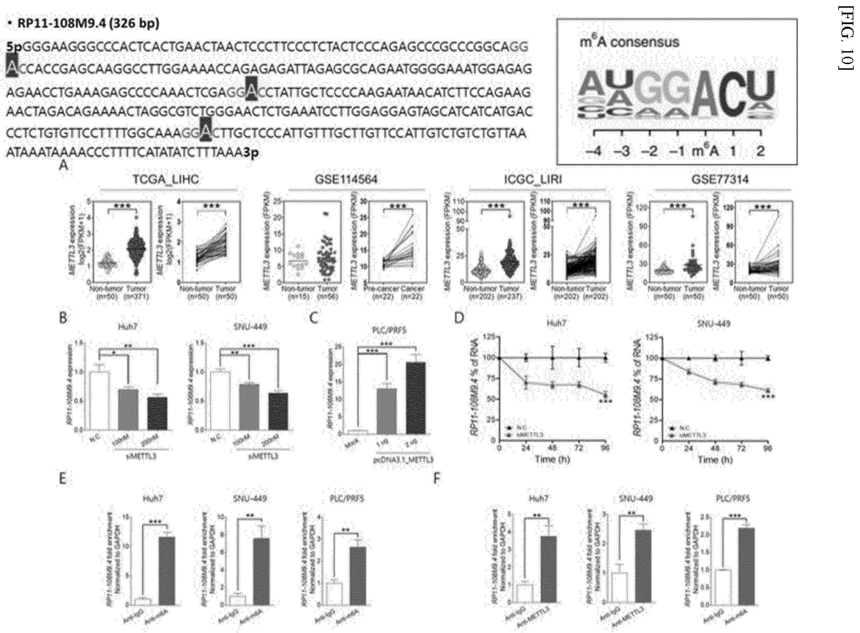

In addition, when qRT-PCR was performed after immunoprecipitation targeting RP11-108M9.4 using an antibody that recognizes N⁶-methyladenosine or METTL3, it was discovered that N⁶-methyladenosine was added to the RP11-108M9.4 transcript, thereby indicating that the abnormal increase in expression of RP11-108M9.4 in liver cancer is due to N⁶-methyladenosine modification of RP11-108M9.4 by METTL3 (FIG. 10).

Liver cancer tissues and surrounding tissues from liver cancer patients who underwent hepatectomy were provided by the Human Genome Resource Center of Ajou University Hospital, and the expression of RP11-108M9.4 and METTL3 was identified by qRT-PCR.

When the expression of RP11-108M9.4 and METTL3 in liver cancer tissues compared to normal surrounding tissues was analyzed by fold change for 86 pairs of patient tissue samples, there were 60 (70%) overexpressions and 16 (19%) downregulated expressions for RP11-108M9.4, and 67 (78%) overexpressions and 13 (15%) downregulated expressions for METTL3.

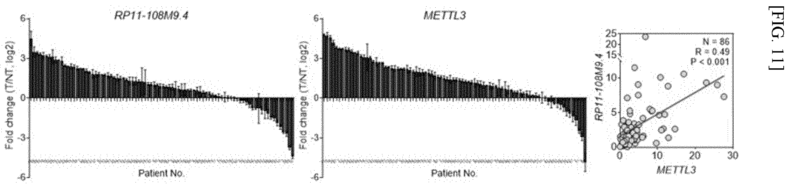

When these expressions were analyzed for correlation, the Pearson r value was 0.49, where P<0.001 is indicated to have a statistically significant positive correlation, suggesting that the abnormal increase in expression of RP11-108M9.4 is associated with the expression of METTL3 in liver cancer patients (FIG. 11).

### <Example 8> Evaluation of diagnostic ability of RP11-108M9.4 as a serum EV liver cancer diagnostic marker

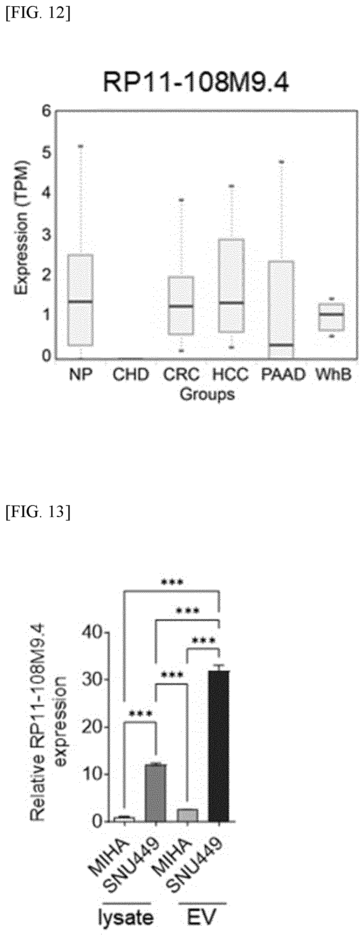

RP11-108M9.4 expression was identified in exosomes using the Exocarta database. In the identification for the expression of RP11-108M9.4 in EVs derived from the blood of patients with various diseases, it was found that RP11-108M9.4 was expressed in EVs derived from the blood of liver cancer patients (FIG. 12).

The expression level of RP11-108M9.4 was determined in cell lines. It was found in cell lysate and EV from MIHA, a normal liver cell line, and SNU449, a liver cancer cell line. It is noted that the expression of RP11-108M9.4 was higher in liver cancer cell lines than in normal liver cell lines in both cell lysates and EVs, and in particular, it was observed to be higher in both normal liver cells and liver cancer cells than in cell lines in EVs (FIG. 13).

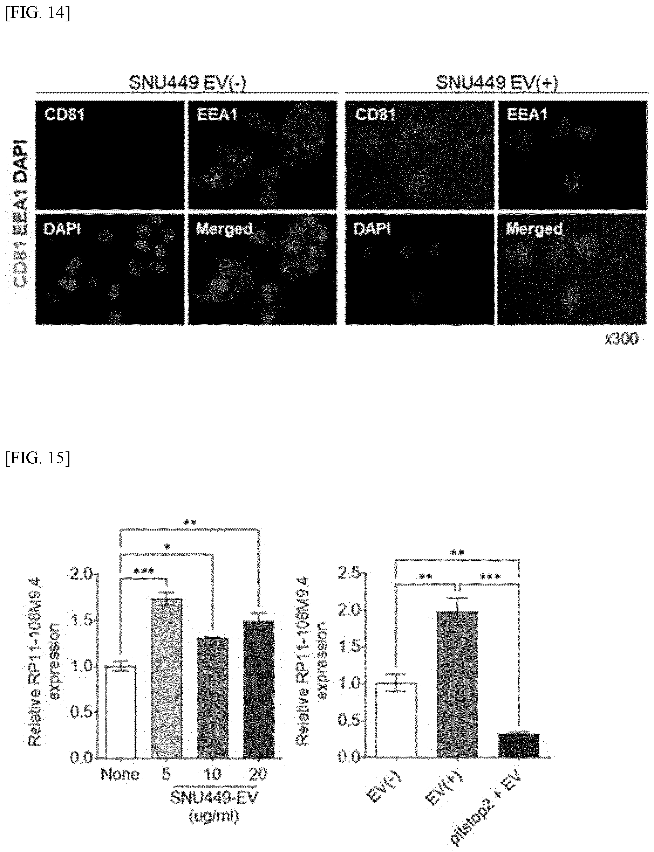

As a result of culturing normal liver cell lines with treatment of liver cancer-derived exosomes, it was found that the red staining area of the EEA1 antibody, an endocytic marker, was stained, overlapping with the green staining area of the CD81 antibody, an exosome marker. This means that exosomes injected from outside were transferred into the cell through endocytosis. In contrast, in the negative control group that was not treated with exosomes, it was found that the CD81 antibody, an exosome marker, was not stained (FIG. 14).

When the expression level of RP11-108M9.4 was observed by treating EVs extracted from the liver cancer cell line SNU449 to the normal liver cell line MIHA, it was observed that the expression of RP11-108M9.4 increased when EVs extracted from the liver cancer cell line SNU449 were injected. In addition, it was determined that RP11-108M9.4 was derived from EV (FIG. 15).

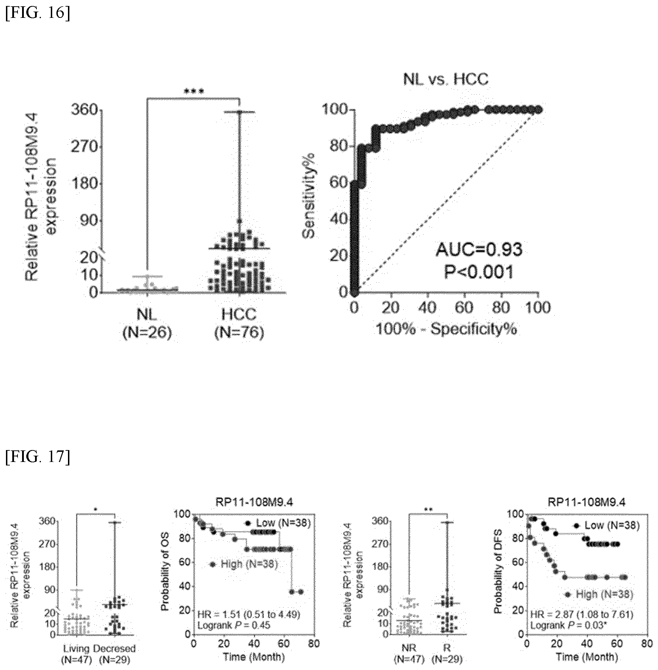

To determine whether RP11-108M9.4 expression in EVs derived from patient serum is higher in the liver cancer as in tissues, as a result of examining the expression of RP11-108M9.4 in the serum of 26 normal patients and serum exosomes of 76 liver cancer patients, the expression of RP11-108M9.4 in serum exosomes was statistically significantly high in liver cancer patients. When the diagnostic ability was evaluated by ROC, the specificity for liver cancer was high with AUC=0.93 (FIG. 16).

To evaluate the clinical significance of the expression level of EV-RP11-108M9.4, the survival rate of liver cancer patients according to survival and recurrence was observed. RP11-108M9.4 was found to have a higher expression in dead patients (Decreased) with liver cancer than in those who survived (Living), and a higher expression in patients with recurrence (R) than in patients with no recurrence (NR), and in the survival analysis, although not statistically significant in OS, patients with relatively high expression of EV-RP11-108M9.4 were evaluated to have a poor prognosis with HR=2.87 and logrank P=0.03 in DFS (FIG. 17).

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

## Claims

1. A biomarker composition for predicting liver cancer recurrence or liver cancer metastasis, comprising long non-coding RNA RP11-108M9.4 as an active ingredient.

2. A biomarker composition for diagnosing a liver cancer, comprising long non-coding RNA RP11-108M9.4 as an active ingredient.

3. The biomarker composition of claim 1 or 2, wherein a transcript of the long non-coding RNA RP11-108M9.4 comprises a GGAC motif which is a variant motif of N⁶-methyladenosine.

4. A composition for predicting liver cancer recurrence or liver cancer metastasis, comprising an agent capable of measuring an expression level of long non-coding RNA RP11-108M9.4 as an active ingredient.

5. A composition for diagnosing a liver cancer, comprising an agent capable of measuring an expression level of long non-coding RNA RP11-108M9.4 as an active ingredient.

6. The composition of claim 4 or 5, wherein the agent capable of measuring the expression level is a primer or probe specifically binding to the long non-coding RNA RP11-108M9.4.

7. The composition of claim 4, wherein the liver cancer metastasis is metastasis from a liver cancer to a lung cancer.

8. A kit for predicting liver cancer recurrence or liver cancer metastasis, comprising the composition according to claim 4.

9. A kit for diagnosing a liver cancer, comprising the composition according to claim 5.

10. A method of providing information necessary for predicting liver cancer recurrence or liver cancer metastasis, the method comprising:
(1) measuring an expression level of long non-coding RP11-108M9.4 from a sample isolated from a liver cancer patient;
(2) comparing the measured expression level of long non-coding RNA RP11-108M9.4 with that of a control sample; and
(3) predicting that there is a high possibility of liver cancer recurrence or liver cancer metastasis if the measured expression level of long non-coding RNA RP11-108M9.4 is higher than that of the control sample.

11. A method of providing information necessary for diagnosing a liver cancer, the method comprising:
(1) measuring an expression level of long non-coding RP11-108M9.4 from a sample isolated from a liver cancer patient;
(2) comparing the measured expression level of long non-coding RNA RP11-108M9.4 with that of a control sample; and
(3) determining as a liver cancer if the measured expression level of long non-coding RNA RP11-108M9.4 is higher than that of the control sample.

12. The method of claim 10 or 11, wherein the increase in the expression level of the long non-coding RNA RP11-108M9.4 is induced by mutation in N⁶-methyladenosine of RP11-108M9.4 by METTL3.

13. The method of claim 11, wherein the sample is a serum exosome.

14. A pharmaceutical composition for preventing or treating liver cancer recurrence or liver cancer metastasis, comprising an expression or activity inhibitor of long non-coding RNA RP11-108M9.4 as an active ingredient.

15. The pharmaceutical composition of claim 14, wherein the expression or activity inhibitor of the long non-coding RNA RP11-108M9.4 is any one selected from the group consisting of antisense nucleotides, small interfering RNA (siRNA), short hairpin RNA (shRNA), compounds, peptides, aptamers, and antibodies that specifically bind to RP11-108M9.4.

16. The pharmaceutical composition of claim 14, wherein the liver cancer metastasis is metastasis from a liver cancer to a lung cancer.

17. A health functional food composition for preventing or attenuating liver cancer recurrence or liver cancer metastasis, comprising an expression or activity inhibitor of long non-coding RNA RP11-108M9.4 as an active ingredient.

18. A method of screening a treatment agent for liver cancer recurrence or liver cancer metastasis, comprising:
(1) contacting isolated liver cancer cells with a test substance;
(2) measuring an expression or activity level of long non-coding RNA RP11-108M9.4 in the liver cancer cells that have come into contact with the test substance; and
(3) selecting a test substance having a reduced expression or activity level of the long non-coding RNA RP11-108M9.4 compared to a control sample.
